# EUROPÄISCHE PATENTSCHRIFT

(11) **EP 2 268 332 B1**
(45) Veröffentlichungstag und Bekanntmachung des Hinweises auf die Patenterteilung: **16.01.2019**
(21) Anmeldenummer: 08735234.0
(22) Anmeldetag: 09.04.2008
(51) Int. Cl.: A61L 29/16, A61L 31/16, A61M 29/02, A61M 25/10

(54) **VERFAHREN ZUR ERZEUGUNG EINER BIOAKTIVEN OBERFLÄCHE AUF EINER ENDOPROTHESE ODER AUF DEM BALLON EINES BALLONKATHETERS**
METHOD FOR PRODUCING A BIOACTIVE SURFACE ON AN ENDOPROSTHESIS OR ON THE BALLOON OF A BALLOON CATHETER
PROCÉDÉ DE PRODUCTION D'UNE SURFACE BIOACTIVE SUR UNE ENDOPROTHÈSE OU SUR LE BALLONNET D'UN CATHÉTER À BALLONNET

(43) Veröffentlichungstag der Anmeldung: 05.01.2011
(73) Patentinhaber: Aachen Scientific International PTE. LTD., 079903 Singapore (SG)
(72) Erfinder: Rübben Alexander, 98000 Monaco (MC)
(74) Vertreter: Schöneborn, Holger
(86) Internationale Anmeldenummer: PCT/EP2008/002960
(87) Internationale Veröffentlichungsnummer: WO 2009/124570

(56) Entgegenhaltungen:
- WO-A-01/52915
- WO-A-02/055122
- WO-A-2006/032904
- US-A- 5 102 402

## Beschreibung

Die vorliegende Erfindung betrifft ein Verfahren zur Erzeugung einer bioaktiven Oberfläche auf dem Ballon eines Ballonkatheters. Die Erfindung betrifft zudem einen Ballon eines Ballonkatheters.

Die sogenannten "minimalinvasiven Verfahren" nehmen in der Medizin einen immer größeren Stellenwert ein. Im Rahmen der Radiologie ist hierbei die interventionelle Radiologie anzusprechen, die wesentlich zur Entwicklung minimalinvasiver Techniken und hierfür notwendiger Geräte und Prothesen aus geeigneten Materialien beigetragen hat. So werden heute kleine Metallgitter als Gefäßendoprothesen, sogenannte Stents, sowohl von Kardiologen als auch von Radiologen in Gefäße eingesetzt, um diese offen zu halten. Bei herkömmlichen Stents kommt es jedoch häufig zu einer Verdickung der Gefäßwand mit konsekutiver Lumeneinengung im Bereich des Stents durch eine Zellproliferation oder durch eine Anlagerung von Zellen. Weiterhin werden Ballonkatheter sowohl von Kardiologen als auch von Radiologen in Gefäße eingesetzt, um diese zu öffnen. Auch bei diesen Eingriffen kann es zu einer Verdickung der Gefäßwand mit konsekutiver Lumeneinengung im Bereich der Aufdehnung durch eine Zellproliferation kommen.

Durch Medikamentenabgabe von der Oberfläche der Endoprothese beziehungsweise von der Oberfläche des Ballons des Ballonkatheters, die zur Verbesserung der Medikamentenbeladung und Medikamentenabgabe mit einer geeigneten polymeren Beschichtung versehen sein kann, kann diesem Problem entgegengewirkt werden. Typischerweise wird ein in einem Lösungsmittel gelöster Wirkstoff auf die Oberfläche der Endoprothese oder auf die Oberfläche des Ballons des Ballonkatheters aufgebracht, wobei das Lösungsmittel anschließend verdunstet. Der Wirkstoff befindet sich dann als Schicht auf der Oberfläche.

Möglichkeiten zur Erzielung einer demgegenüber verbesserten Haftung des Wirkstoffes auf der Oberfläche werden beispielsweise in den Dokumenten US 5,102,402 und US 6,129,705 beschrieben. In dem Dokument US 5,102,402 ist ein mit Medikamenten beschichteter Ballonkatheter beschrieben. Dabei werden in einer ersten Variante mit einem Wirkstoff bzw. Medikament gefüllte Mikrokapseln von Falten in der Ballonoberfläche umschlossen und so mechanisch in der jeweiligen Position gehalten. In einer zweiten Variante sind die Mikrokapseln mit Hilfe eines Haftvermittlers auf die Ballonoberfläche geklebt. Im Rahmen dieses Dokuments werden auch unbeschichtete Wirkstoffkristalle als Mikrokapseln angesehen.

In dem Dokument US 6,129,705 werden ein Ballonkatheter und ein Stent beschrieben, deren Oberflächen jeweils eine Beschichtung aufweisen, in die mit einem Wirkstoff gefüllte Mikrokapseln vollständig eingebettet sind. In einer Ausführungsvariante wird ein Ballonkatheter beschrieben, in dessen unbeschichtete Oberfläche mit einem Wirkstoff gefüllte Mikrokapseln während des Herstellungsprozesses extrudiert worden sind. Allerdings handelt es sich bei dem Einfüllen des Wirkstoffes in Mikrokapseln und dem anschließenden Befestigen bzw. Einbetten der Mikrokapseln auf der Ballon- bzw. Stentoberfläche um vergleichsweise aufwendige und damit kostenintensive Verfahren.

In der WO 01/52915 A1 wird ein Implantat, insbesondere ein Stent, offenbart, das ein Coating aus einer wasserquellbaren Polymermatrix aufweist. Dabei kann ein anionischer Wirkstoff, insbesondere eine Nucleinsäure, auf die Oberfläche aufgebracht werden, indem ein Lösungsmittel verwendet wird, das das Polymer aufquellen lässt. Das Lösungsmittel ist dabei typischerweise wässriger Natur.

In der WO 02/055122 A1 wird ein Verfahren zur Herstellung eines wirkstoffbeladenen Stents beschrieben, bei dem ein Metallstent mit Polymer beschichtet wird und der beschichtete Stent mit einem Wirkstoff in einem organischen Lösungsmittel, welches das Polymer quellen kann, kontaktiert und das Lösungsmittel verdampft wird.

In der WO 2006/032904 A2 werden Implantate beschrieben, die mit Hilfe eines Quellmittels und Triclosan imprägniert werden. Es handelt sich bei den Implantaten aber weder um Endoprothesen noch um Ballonkatheter.

Es ist daher eine erste Aufgabe der vorliegenden Erfindung, ein vorteilhaftes Verfahren zur Erzeugung einer bioaktiven Oberfläche auf dem Ballon eines Ballonkatheters zur Verfügung zu stellen. Es ist eine zweite Aufgabe der Erfindung, einen vorteilhaften Ballon eines Ballonkatheters zur Verfügung zu stellen.

Die erste Aufgabe wird durch ein Verfahren nach Anspruch 1, die zweite Aufgabe durch einen Ballon eines Ballonkatheters nach Anspruch 11 gelöst. Die abhängigen Ansprüche enthalten weitere vorteilhafte Ausgestaltungen der Erfindung. Die Merkmale sind sowohl einzeln als auch in Kombination miteinander vorteilhaft.

In dem erfindungsgemäßen Verfahren zur Erzeugung einer bioaktiven Oberfläche auf dem Ballon eines Ballonkatheters wird die Oberfläche des Ballons angeweicht und mit einer Lösung eines Wirkstoffes benetzt. Anschließend wird das Lösungsmittel von dem Wirkstoff getrennt.

Dabei handelt es sich um ein sehr einfaches und kostengünstiges Verfahren, bei dem der Wirkstoff infolge des Anweichens der Oberfläche ganz oder teilweise in die Oberfläche eingebettet wird bzw. an der Oberfläche anhaftet. Im Rahmen des erfindungsgemäßen Verfahrens wird das Prinzip des Quellschweißens (solvent welding) genutzt. Das erfindungsgemäße Verfahren bewirkt im Vergleich zu einem bloßen Abscheiden des Wirkstoffes auf der Oberfläche eine verbesserte Haftung des Wirkstoffes an bzw. in der Oberfläche. Zudem handelt es sich um ein im Vergleich zu den in den Dokumenten US 5,102,402 und US 6,129,705 beschrieben Verfahren um ein erheblich einfacheres und kostengünstigeres Verfahren.

Die Oberfläche des Ballons wird mit Hilfe der Lösung des Wirkstoffes angeweicht. Als Lösungsmittel können zum Beispiel Dimethylsulfoxid (DMSO), Dioxan, Dimethylformamid (DMF), Tetrahydrofuran (THF), Methylenchlorid oder Chloroform verwendet werden. Diese Lösungsmittel sind mit Wasser mischbar.

Dadurch, daß die Oberfläche des Ballons mit Hilfe der Lösung des Wirkstoffes angeweicht wird, können das Anweichen und das Benetzen gleichzeitig durchgeführt werden. Die Oberfläche des Ballons kann beispielsweise durch Eintauchen, Besprühen oder Pipettieren mit der Lösung des Wirkstoffes benetzt werden.

Es wird ein Ballon verwendet, dessen Oberfläche ein Polymer, nämlich Nylon oder ein Ny-Ion-Pebax-Gemisch umfaßt.

Der verwendete Wirkstoff kann insbesondere Tretinoin und/oder Orphanrezeptoragonisten und/oder Corticosteroide und/oder Steroidhormone und/oder Paclitaxel und/oder Taxol und/oder Rapamune und/oder Tacrolimus und/oder hydrophobe Proteine und/oder zellproliferationsverändernde Substanzen umfassen. Als Steroidhormone können beispielsweise Methylprednisolon, Dexamethason oder Östradiol verwendet werden.

Vorteilhafterweise kann überflüssiger Wirkstoff und Lösungsmittel durch ein Bewegen des Ballons, zum Beispiel Abschleudern oder Abschlagen, von der benetzten Oberfläche entfernt werden. Durch das Bewegen des Ballons, insbesondere durch Schleudern, wird eine gleichmäßige Verteilung des Lösungsmittels und damit auch des Wirkstoffes auf der Oberfläche des Ballons erreicht. Dies beugt einer möglichen Bildung von Klumpen effektiv vor.

Weiterhin kann die Oberfläche des Ballons vor dem Anweichen vergrößert werden. Beispielsweise kann die Oberfläche des Ballons mechanisch, thermisch oder chemisch vergrößert werden. Die Oberfläche des Ballons kann insbesondere durch Strukturieren oder Profilieren vergrößert werden. Beispielsweise kann die Oberfläche des Ballons durch Aufrauen strukturiert oder profiliert werden. Vorteilhafterweise werden durch das Vergrößern der Oberfläche des Ballons auf der Oberfläche Vertiefungen mit einer Tiefe von 5-50 µm und einer Breite von 5-50 µm erzeugt.

Die Oberfläche des Ballons wird mit einer Lösung eines Wirkstoffes in einem mit Wasser mischbaren Lösungsmittel benetzt, wobei der Wirkstoff eine Löslichkeit von maximal 0,9 mg/ml in destilliertem Wasser hat. Das Lösungsmittel kann von dem Wirkstoff getrennt werden, indem der benetzte Ballon in Wasser getaucht wird. Dabei fällt der in Wasser unlösliche Wirkstoff aus und scheidet sich teilweise auf der Oberfläche des Ballons ab. Da die Oberfläche des Ballons angeweicht ist, wird der Wirkstoff ganz oder teilweise in die Oberfläche eingebettet oder haftet zumindest an der Oberfläche an. Als wasserlösliche Lösungsmittel können insbesondere die oben genannten Lösungsmittel Verwendung finden.

Der erfindungsgemäße Ballon eines Ballonkatheters umfaßt eine unbeschichtete Oberfläche. Ein ungekapselter Wirkstoff ist zumindest partiell in das Material der Oberfläche eingebettet. Der Wirkstoff kann also ganz oder teilweise in die Oberfläche eingebettet sein. Auch im Falle einer nur teilweisen Einbettung haftet der Wirkstoff zumindest an der Oberfläche an.

Das Material der Oberfläche des Ballons umfaßt Nylon oder ein Nylon-Pebax-Gemisch. Der Ballon kann auch aus Nylon oder einem Nylon-Pebax-Gemisch bestehen.

Der zumindest partiell in die Oberfläche eingebettete Wirkstoff kann Tretinoin und/oder Orphanrezeptoragonisten und/oder Corticosteroide und/oder Steroidhormone und/oder Paclitaxel und/oder Taxol Taxolderivate und/oder Rapamune und/oder Tacrolimus und/oder hydrophobe Proteine und/oder zellproliferationsverändernde Substanzen umfassen. Die Steroidhormone können beispielsweise Methylprednisolon, Dexamethason oder Östradiol umfassen.

Der erfindungsgemäße Ballonkatheter umfaßt einen erfindungsgemäßen Ballon, wie er zuvor beschrieben wurde. Sowohl der erfindungsgemäße Ballon als auch der erfindungsgemäße Ballonkatheter haben den Vorteil, daß sie einfach und kostengünstig hergestellt werden können und zudem eine sichere Haftung des Wirkstoffes auf der Oberfläche infolge der zumindest partiellen Einbettung desselben gewährleisten.

Weitere Merkmale, Eigenschaften und Vorteile der vorliegenden Erfindung werden nachfolgend anhand von Ausführungsbeispielen unter Bezugnahme auf die beiliegenden Figuren beschrieben. Die Merkmale sind sowohl einzeln als auch in Kombination miteinander vorteilhaft.
Fig. 1 zeigt schematisch einen Ballonkatheter, der mit der Lösung eines Wirkstoffes besprüht wird.
Fig. 2 zeigt schematisch einen Schnitt durch einen Teil der Oberfläche des Ballons eines Ballonkatheters, auf dessen angeweichter Oberfläche sich die Lösung eines Wirkstoffes befindet.
Fig. 3 zeigt schematisch einen Schnitt durch einen Teil der Oberfläche des Ballons eines Ballonkatheters, in dessen Oberfläche ein Wirkstoff eingebettet ist.
Fig. 4 zeigt schematisch das Eintauchen eines mit der Lösung eines Wirkstoffes benetzten Ballonkatheters in ein Wasserbad.
Fig. 5 zeigt schematisch einen Schnitt durch einen Teil der sich im Wasser befindlichen angeweichten Oberfläche des Ballons des Ballonkatheters.
Fig. 6 zeigt schematisch einen Schnitt durch einen Teil der Oberfläche eines Ballons eines Ballonkatheters, in dessen aufgeraute Oberfläche ein Wirkstoff eingebettet ist.

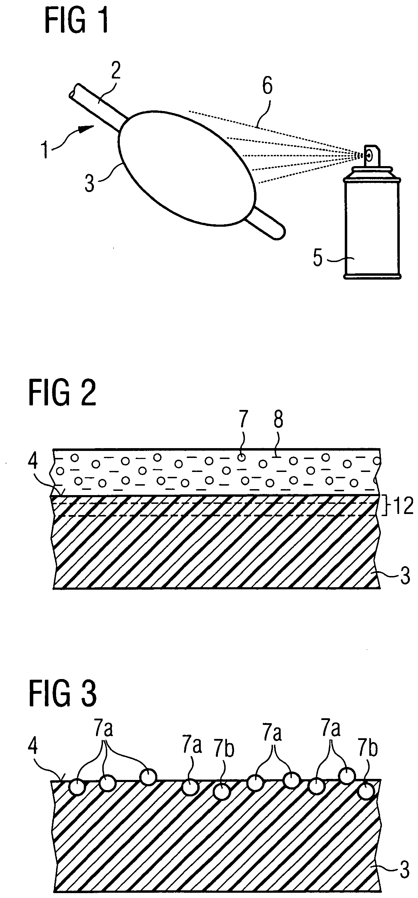

Ein Ausführungsbeispiel der vorliegenden Erfindung wird nachfolgend anhand der Figuren 1 bis 6 näher beschrieben. Die Figur 1 zeigt schematisch einen Ballonkatheter 1, der mithilfe einer Sprühvorrichtung 5 mit der Lösung 6 eines Wirkstoffes 7 in einem mit Wasser mischbaren Lösungsmittel 8 besprüht wird. Der Ballonkatheter 1 umfaßt eine Kathetersonde 2 und einen Ballon 3. Der Ballon 3 umschließt einen Teil der Kathetersonde 2. Der Ballon 3 besteht aus Nylon oder einem Nylon-Pebax-Gemisch. Die Oberfläche des Ballons 3 wird mithilfe der Sprühvorrichtung 5 mit einer Lösung 6 eines Wirkstoffes 7 in einem mit Wasser mischbaren Lösungsmittel 8 besprüht und auf diese Weise benetzt. Anstelle des Besprühens kann der Ballon 3 des Ballonkatheters 1 auch in die Lösung eines Wirkstoffes in einem mit Wasser mischbaren Lösungsmittel eingetaucht oder mit dieser pipettiert werden. Das Lösungsmittel 8 bewirkt, daß die Oberfläche des Ballons 3 angeweicht wird.

Die Figur 2 zeigt schematisch einen Schnitt durch einen Teil der Oberfläche des Ballons 3 des Ballonkatheters 1, dessen der Oberfläche mit der Lösung eines Wirkstoffes 7 benetzt wurde. Der durch das Lösungsmittel 8 angeweichte Bereich der Oberfläche 4 ist mit der Bezugsziffer 12 gekennzeichnet. Auf der angeweichten Oberfläche 4 des Ballons 3 befindet sich nun eine Mischung aus einem Lösungsmittel 8 und einem Wirkstoff 7.

Bei dem verwendeten Wirkstoff 7 kann es sich beispielsweise um Tretinoin und/oder Orphanrezeptoragonisten und/oder Corticosteroide und/oder Steroidhormone und/oder Paclitaxel und/oder Taxol und/oder Rapamune und/oder Tacrolimus und/oder hydrophobe Proteine und/oder zellproliferationsverändernde Substanzen handeln, wobei als Steroidhormone Methylprednisolon, Dexamethason oder Östradiol verwendet werden können. Bei dem Lösungsmittel 8 kann es sich beispielsweise um Dimethylsulfoxid (DMSO), Dioxan, Dimethylformamid (DMF), Tetrahydrofuran (THF), Methylenchlorid oder Chloroform handeln.

Die Figur 3 zeigt einen Schnitt durch einen Teil der Oberfläche 4 eines Ballons 3 eines Ballonkatheter 1, in dessen Oberfläche 4 ein Wirkstoff 7a, 7b eingebettet ist. Der teilweise in die Oberfläche 4 eingebettete Wirkstoff ist mit der Bezugsziffer 7a gekennzeichnet und der vollständig in die Oberfläche 4 eingebettete Wirkstoff ist mit der Bezugsziffer 7b gekennzeichnet.

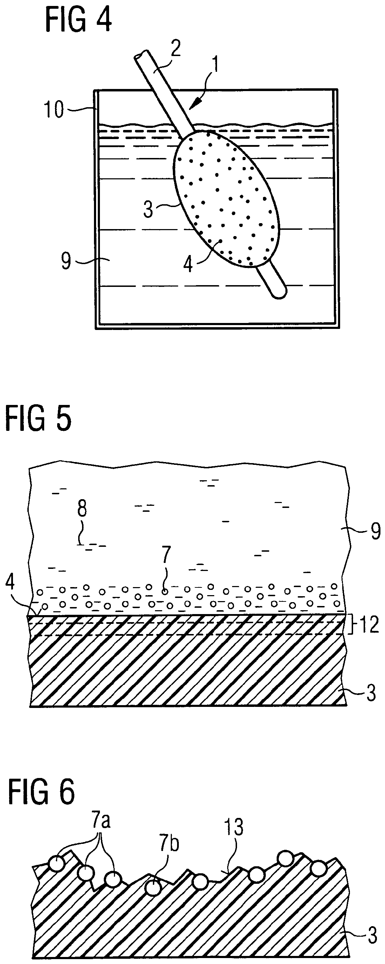

Die Erfindung wird im Folgenden anhand der Figuren 4 und 5 näher beschrieben. In dieser Ausführungsvariante wird der Ballon 3 zunächst, wie im Zusammenhang mit den Figuren 1 und 2 beschrieben, angeweicht und mit einer Lösung eines Wirkstoffes 6 benetzt. Anschließend wird der Wirkstoff 7 durch Ausfällung auf der angeweichten Oberfläche 4 abgeschieden.

Die Figur 4 zeigt schematisch das Eintauchen des mit der Lösung eines Wirkstoffes 6 benetzten Ballonkatheters 1 in ein Wasserbad. In der Figur 4 ist ein Gefäß 10 gezeigt, welches mit Wasser 9 gefüllt ist. Der Ballon 3 des Ballonkatheters 1 ist vollständig in das Wasser 9 eingetaucht. Die angeweichte und benetzte Oberfläche 4 des Ballons 3 kommt dabei direkt mit dem Wasser 9 in Kontakt.

Die Figur 5 zeigt schematisch einen Schnitt durch einen Teil der sich im Wasser 9 befindlichen angeweichten Oberfläche 4 des Ballons 3 des Ballonkatheters 1. Infolge des direkten Kontaktes zwischen dem Wasser 9 und dem mit Wasser mischbaren Lösungsmittel 8 tritt eine zunehmende Vermischung des Lösungsmittels 8 mit dem Wasser 9 auf. Dies ist in der Figur 5 schematisch angedeutet. Der im Wasser unlösliche oder zumindest nur schwer lösliche Wirkstoff 7 fällt hingegen auf der angeweichten Oberfläche 4 des Ballons 3 aus und wird in diese, wie im Zusammenhang mit der Figur 3 beschieben, eingebettet. Auf diese Weise wird der Wirkstoff 7 von dem Lösungsmittel 8 getrennt.

Nachdem der Wirkstoff 7 auf der Oberfläche 4 ausgefallen ist, kann der Ballon 3 des Ballonkatheters 1 aus dem Wasserbad entfernt werden. Das möglicherweise auf der Oberfläche 4 des Ballons 3 noch befindliche Wasser kann anschließend verdunsten gelassen werden. Allerdings ist infolge der Einbettung des Wirkstoffes 7 in die Oberfläche 4 der Wirkstoff 7 ohnehin bereits fest mit der Oberfläche 4 des Ballons 3 verbunden, so daß der Ballon 3 im Prinzip nicht getrocknet werden muss, also auch naß bleiben kann.

Im Folgenden wird eine dritte Ausführungsvariante des ersten Ausführungsbeispiels anhand der Figur 6 näher beschrieben. In dieser Ausführungsvariante wird die Oberfläche 4 des Ballons 3 vor dem Anweichen zunächst aufgeraut. Dies kann insbesondere mechanisch, chemisch oder thermisch erfolgen. Dabei können auf der Oberfläche 4 des Ballons 3 insbesondere Vertiefungen mit einer Tiefe von 5-50 µm und einer Breite von 5-50 µm erzeugt werden.

Anschließend wird die aufgeraute Oberfläche 13 des Ballons 3, wie in den ersten beiden Ausführungsvarianten beschrieben, angeweicht und mit der Lösung eines Wirkstoffes 6 benetzt. Nachdem das Lösungsmittel 8 durch Ausfällen von dem Wirkstoff 7 getrennt wurde, ist der Wirkstoff 7 ganz oder teilweise in die aufgeraute Oberfläche 13 des Ballons 3 eingebettet. Dies ist in der Figur 6 schematisch dargestellt.

Die Figur 6 zeigt schematisch einen Schnitt durch einen Teil der Oberfläche 13 eines Ballons 3 eines Ballonkatheters 1, in dessen aufgeraute Oberfläche 13 ein Wirkstoff 7 eingebettet ist. Der teilweise in die Oberfläche 13 eingebettete Wirkstoff ist mit der Bezugsziffer 7a gekennzeichnet, der vollständig eingebettete Wirkstoff ist mit der Bezugsziffer 7b gekennzeichnet.

## Patentansprüche

1. Verfahren zur Erzeugung einer bioaktiven Oberfläche auf dem Ballon eines Ballonkatheters (1), umfassend
- einen Schritt, in dem die Oberfläche (4) des Ballons (3) mit einer einen Wirkstoff (7) und ein mit Wasser mischbares Lösungsmittel (8) umfassenden Lösung (6) benetzt wird, wobei die Oberfläche (4) des Ballons (3) mit Hilfe des Lösungsmittels (8) in der Lösung (6) angeweicht wird,
- und einen Schritt, in dem das Lösungsmittel (8) von dem Wirkstoff (7) getrennt wird,
wobei ein unbeschichteter Ballon (3) verwendet wird, dessen Oberfläche (4) Nylon oder ein Nylon-Pebax-Gemisch umfasst, und der Wirkstoff (7) in destilliertem Wasser eine Löslichkeit von maximal 0,9 mg/ml hat.

2. Verfahren nach Anspruch 1, worin als Lösungsmittel (8) Dimethylsulfoxid (DMSO), Dioxan, Dimethylfomamid (DMF), Tetrahydrofuran (THF), Methylenchlorid oder Chloroform verwendet wird.

3. Verfahren nach Anspruch 1 oder 2, worin der verwendete Wirkstoff (7) Tretinoin und/oder Orphanrezeptoragonisten und/oder Corticosteroide und/oder Steroidhormone und/oder Paclitaxel und/oder Taxol und/oder Rapamune und/oder Tacrolismus und/oder hydrophobe Proteine und/oder zellproliferationsverändernde Substanzen umfasst.

4. Verfahren nach Anspruch 3, worin als Steroidhormone Methylprednisolon, Dexamethason oder Östradiol verwendet werden.

5. Verfahren nach einem der Ansprüche 1 bis 4, worin die Oberfläche (4) des Ballons (3) vor dem Anweichen vergrößert wird.

6. Verfahren nach Anspruch 5, worin die Oberfläche (4) des Ballons (3) mechanisch, thermisch oder chemisch vergrößert wird.

7. Verfahren nach Anspruch 6, worin die Oberfläche (4) des Ballons (3) durch Strukturieren oder Profilieren vergrößert wird.

8. Verfahren nach Anspruch 7, worin die Oberfläche (4) des Ballons (3) durch Aufrauen strukturiert oder profiliert wird.

9. Verfahren nach einem der Ansprüche 5 bis 8, worin durch das Vergrößern der Oberfläche (4) des Ballons (3) auf der Oberfläche (4) Vertiefungen mit einer Tiefe von 5-50 µm und einer Breite von 5-50 µm erzeugt werden.

10. Verfahren nach einem der Ansprüche 1 bis 9, worin das Lösungsmittel (8) von dem Wirkstoff (7) getrennt wird, indem der benetzte Ballon (3) in Wasser (9) getaucht wird, wobei der in Wasser unlösliche Wirkstoff (7) ausfällt und sich teilweise auf der Oberfläche (4) abscheidet.

11. Ballon (3) eines Ballonkatheters (1) mit einer unter Verwendung eines Verfahrens nach einem der Ansprüche 1 bis 10 erzeugten bioaktiven Oberfläche, wobei der Ballon (3) vor Anwendung des Verfahrens unbeschichtet ist und eine Oberfläche aufweist, die Nylon oder ein Nylon-Pebax-Gemisch umfasst.

## Claims

1. Method for generating a bioactive surface on the balloon of a balloon catheter (1), comprising
- a step in which the surface (4) of the balloon (3) is wetted with a solution (6) comprising an active ingredient (7) and a water-miscible solvent (8), the surface (4) of the balloon (3) being soaked in the solution (6) with the aid of the solvent (8),
- and a step in which the solvent (8) is separated from the active ingredient (7),
an uncoated balloon (3) being used, the surface (4) of which comprises nylon or a nylon-Pebax blend, and the active ingredient (7) having a solubility of not more than 0.9 mg/ml in distilled water.

2. Method according to Claim 1, wherein the solvent (8) used is dimethyl sulfoxide (DMSO), dioxane, dimethylformamide (DMF), tetrahydrofuran (THF), methylene chloride or chloroform.

3. Method according to Claim 1 or 2, wherein the active ingredient (7) used comprises tretinoin and/or orphan receptor agonists and/or corticosteroids and/or steroid hormones and/or paclitaxel and/or taxol and/or rapamune and/or tacrolimus and/or hydrophobic proteins and/or cell proliferation-altering substances.

4. Method according to Claim 3, wherein the steroid hormones used are methylprednisolone, dexamethasone or oestradiol.

5. Method according to any of Claims 1 to 4, wherein the surface (4) of the balloon (3) is enlarged before soaking.

6. Method according to Claim 5, wherein the surface (4) of the balloon (3) is enlarged mechanically, thermally or chemically.

7. Method according to Claim 6, wherein the surface (4) of the balloon (3) is enlarged by structuring or profiling.

8. Method according to Claim 7, wherein the surface (4) of the balloon (3) is structured or profiled by roughening.

9. Method according to any of Claims 5 to 8, wherein the enlargement of the surface (4) of the balloon (3) generates on the surface (4) indentations having a depth of 5-50 µm and a width of 5-50 µm.

10. Method according to any of Claims 1 to 9, wherein the solvent (8) is separated from the active ingredient (7) by the wetted balloon (3) being immersed in water (9), with the water-insoluble active ingredient (7) precipitating and being deposited in part on the surface (4).

11. Balloon (3) of a balloon catheter (1) having a bioactive surface generated using a method according to any of Claims 1 to 10, the balloon (3) being uncoated before use of the method and having a surface comprising nylon or a nylon-Pebax blend.

## Revendications

1. Procédé pour générer une surface bioactive sur le ballonnet d'un cathéter à ballonnet (1), comprenant
- une étape dans laquelle on mouille la surface (4) du ballonnet (3) avec une solution (6) comprenant une substance active (7) et un solvant (8) miscible à l'eau, la surface (4) du ballonnet (3) étant détrempée à l'aide du solvant (8) dans la solution (6),
- et une étape dans laquelle on sépare le solvant (8) de la substance active (7),
en utilisant un ballonnet (3) non revêtu, dont la surface (4) comprend du Nylon ou un mélange de Nylon et Pebax, et la substance active (7) a une solubilité dans l'eau distillée d'au maximum 0,9 mg/ml.

2. Procédé selon la revendication 1, dans lequel on utilise comme solvant (8) le diméthylsulfoxyde (DMSO), le dioxane, le diméthylformamide (DMF), le tétrahydrofurane (THF), le chlorure de méthylène ou le chloroforme.

3. Procédé selon la revendication 1 ou 2, dans lequel la substance active (7) utilisée comprend la trétinoïne et/ou des antagonistes des récepteurs orphelins et/ou des corticostéroïdes et/ou des hormones stéroïdiennes et/ou le paclitaxel et/ou le Taxol et/ou le Rapamune et/ou le tacrolimus et/ou des protéines hydrophobes et/ou des substances modifiant la prolifération cellulaire.

4. Procédé selon la revendication 3, dans lequel on utilise comme hormones stéroïdiennes la méthylprednisolone, la dexaméthasone ou l'oestradiol.

5. Procédé selon l'une quelconque des revendications 1 à 4, dans lequel avant le détrempage on agrandit la surface (4) du ballonnet (3).

6. Procédé selon la revendication 5, dans lequel on agrandit la surface (4) du ballonnet (3) par un moyen mécanique, thermique ou chimique.

7. Procédé selon la revendication 6, dans lequel on agrandit la surface (4) du ballonnet (3) par structuration ou profilage.

8. Procédé selon la revendication 7, dans lequel la surface (4) du ballonnet (3) est profilée ou structurée par rugosification.

9. Procédé selon l'une quelconque des revendications 5 à 8, dans lequel par agrandissement de la surface (4) du ballonnet (3) on engendre sur la surface (4) des creux ayant une profondeur de 5-50 µm et une largeur de 5-50 µm.

10. Procédé selon l'une quelconque des revendications 1 à 9, dans lequel on sépare le solvant (8) de la substance active (7) en plongeant dans de l'eau (9) le ballonnet (3) mouillé, de sorte que la substance active (7) insoluble dans l'eau précipite et se dépose en partie sur la surface (4).

11. Ballonnet (3) d'un cathéter à ballonnet (1) comportant une surface bioactive engendrée avec utilisation d'un procédé selon l'une quelconque des revendications 1 à 10, le ballonnet (3) étant non revêtu avant l'application du procédé et présentant une surface qui comprend du Nylon ou un mélange de Nylon et Pebax.
